# EUROPEAN PATENT SPECIFICATION

(11) **EP 0 638 169 B1**
(45) Date of publication and mention of the grant of the patent: **30.07.1997**
(21) Application number: 93912008.5
(22) Date of filing: 27.04.1993
(51) Int. Cl.: G01N 33/18

(54) **A METHOD AND DEVICE FOR THE DETECTION OF THE DISCHARGE OF HYDROCARBONS IN LIQUID FORM, IN WATER, ON THE GROUND OR IN THE SUBSURFACE**
VERFAHREN UND VORRICHTUNG ZUM NACHWEIS DER ABFUHR VON SICH IN FLÜSSIGEM ZUSTAND BEFINDLICHEN KOHLENWASSERSTOFFEN IN WASSER, AUF DEN BODEN ODER IN DIE BODENSCHICHTEN UNTER DER OBENEN BODENSCHICHT
PROCEDE ET DISPOSITIF DE DETECTION DE DECHARGE D'HYDROCARBURES A L'ETAT LIQUIDE DANS L'EAU, SUR LE SOL OU DANS LE SOUS-SOL

(30) Priority: 29.04.1992 NO 921669
(43) Date of publication of application: 15.02.1995
(73) Proprietor: MOESTUE, Clarin, N-1342 Jar (NO)
(72) Inventor: MOESTUE, Clarin, N-1342 Jar (NO)
(74) Representative: Popp, Eugen, Dr.
(86) International application number: NO9300067
(87) International publication number: WO9322671

(56) References cited:
- EP-A- 0 295 911
- EP-A- 0 337 630
- GB-A- 1 402 825
- US-A- 3 733 594
- US-A- 4 131 773
- US-A- 4 223 552
- US-A- 4 563 674
- US-A- 4 860 573

## Description

The invention concerns a method for the detection of hydrocarbons in liquid form at normal ambient temperature, in water, on the ground or in the subsurface, especially for the detection of oil spillage on water or in ground water. The invention also concerns a detector device for the execution of the method according to the invention.

The discharge and spillage of hydrocarbons, above all oil, into the natural surroundings is considered to be a serious problem, with grave consequences for the environment when it takes place. The risk of this kind of accident happening is very great, due to the wide-spread use of hydrocarbons in liquid form in practically every area where mankind travels and lives. The danger of discharge is particularly great during the storage and transport of hydrocarbons. Examples of this kind of discharge can be the leakage of oil from tanks and pipelines into the surface of the ground or the subsurface, thus causing the oil to be deposited in the ground water, or discharges during loading and unloading of vessels and vehicles or in connection with transport of these. Finally, oil spillage in surface water or on the sea is one of the major environmental problems of our time, e.g. in connection with shipwrecks or discharges in connection with oil production at sea.

In order to be able to issue a warning concerning the discharge of hydrocarbons and oil spillage, a number of methods have been developed, both for in-situ detection and remote surveillance. These methods are based on the use of more or less expensive instrumentation or are designed for periodic surveillance of vulnerable areas, e.g. from the air.

The United States Patent US-A-3,733,594 discloses an alarm apparatus for detecting and indicating oil slicks. The apparatus incorporates a hydrophobic oil absorbent material which floats on the surface of the water. On contact with oil of an oil slick, the hydrophobic material absorbs oil and sinks into the water. Upon sinking to a predetermined depth, an alarm signalling device is actuated thereby indicating the presence of an oil slick.

The European Patent Application EP-A-0,337,630 discloses an oil leak detecting apparatus comprising an elongated oil sensor supported in a protective tube, which is open at its bottom end for the ingress of leaking oil. The sensor is encased in a sheath of non-woven fabric made of an absorbing material such as isotactic polypropylene. When oil penetrates the sensor, an increase in the electrical resistance thereof is detected.

The United States Patent US-A-4,563,674 discloses a floating sensor for detection of oil leaks at a water surface. A cloth layer is affixed to the outside of the detecting device so as to form a boundary of water at a height above the general surface level adjacent to the apparatus. The cloth layer is made of a sheet of woven or non-woven fabric made of threads having a large or small water absorption, as desired. The detecting device itself absorbs the leakage oil and comprises warning means which operate according to a change in electrical conductivity caused by the absorption of oil.

The United States Patent US-A-4,223,552 discloses an apparatus for detecting hydrocarbon spills on a water surface. A sensing element is embedded in an absorbent material having an affinity for the liquid hydrocarbon. A detection circuit measures an electrical property of the sensing element. The sensing element is embedded in the absorbent material at a predetermined distance from the surface of the water level being monitored.

Furthermore GB-A-1 402 825 discloses a method and device for the determination of the concentration of small amounts of oil in water, wherein the oil is concentrated from oily water into a filter material which absorbs oil and the amount of oil absorbed is measured either by a photoelectric method or a dielectric method. However, the disclosure relies on an active absorption method and presupposes an already detected oil spill, before proceeding to the concentration measurement of a locally acquired sample. It is not suitable for instant and automatic detection of possible oil spills over a wide area, e.g. by means of distributed, passive, simple and inexpensive detectors.

However, there is a need for an improved detector which is inexpensive to produce and which at the same time can be used for permanent surveillance of areas which are threatened by hydrocarbon discharge such as oil spillage.

Thus the object of the present invention is to develop a simple and inexpensive detection principle and thereafter to use this detection principle in a simple detector device which permits the detection of the discharge of hydrocarbons in liquid form both at sea, on the ground and in the subsurface.

The above-mentioned objects are achieved with a method as defined in claim 1 and by a detector device according to the invention as defined in claim 11. Further embodiments of the method and the detector according to the invention are presented in the independent claims 2-10 and the independent claims 12-21 respectively.

The invention will now be explained in more detail in connection with embodiments of the detector taken in conjunction with the accompanying drawing.

Fig. 1 is a detector device according to an embodiment of the invention, based on the use of ultrasound.

Fig. 2 is a detector device according to an embodiment of the invention, based on/the use of light reflection.

Fig. 3 is another embodiment of a detector device based on the use of light reflection.

Fig. 4 is a detector device according to an embodiment based on the measurement of the dielectric constant.

Fig. 5 is a detector device according to an embodiment based on the measurement of buoyancy.

Fig. 6 is a detector device according to an embodiment based on a combination of two different detection principles.

Fig. 7 is a detector device used for the detection of leakage from a buried oil tank.

The method and the detector device according to the present invention are based on the use of a material which has the ability to attract hydrocarbons in liquid form, e.g. oil or other chemicals with a hydrocarbon basis and which exist in liquid form, while at the same time the material does not absorb or become moistened by water. A material of this kind is used in an absorbent body, and by absorbing hydrocarbons such as oil, certain of its physical parameters will change.

As the material it is preferred to use a polypropylene cloth of the type "ERGON Sorb" supplied by HP Textiles, Oldenzaal, Holland. "ERGON Sorb" is a so-called "melt-blown" polypropylene cloth which is supplied in whit in different thicknesses. The cloth can absorb 11.2 times its own weight in oil, but only attracts 0.023 times its own weight in water in the course of 15 minutes. Consequently "ERGON Sorb" has a relative affinity oil/water of 11.2/0.023 or a selective absorption capacity for oil which is 487 times greater than that for water. If the "ERGON sorb" cloth is placed under water pressure or compressed under water, it will absorb some water, but the cloth will not become saturated, nor will the cloth's ability to attract hydrocarbons such as oil be destroyed.

It should be understood, however, that other materials of a similar kind which demonstrate such a high selective absorption capacity with regard to hydrocarbons such as oil can also be utilized. Consequently, the embodiment using the "ERGON Sorb" cloth is only intended as an example of a preferred embodiment of the detector device according to the invention and is not intended to limit this in any way.

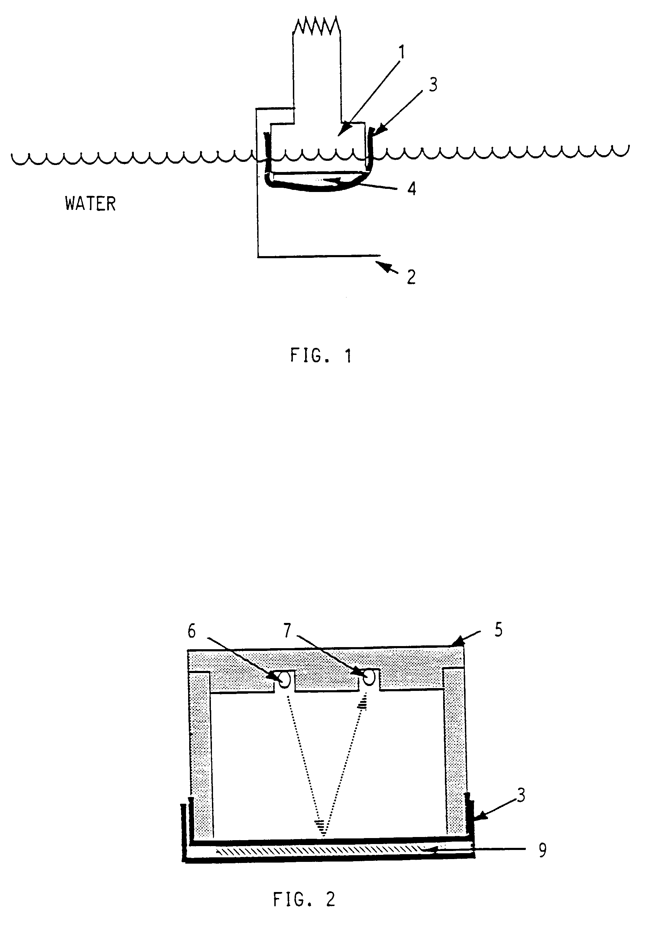

Fig. 1 shows a detector device based on the use of ultrasound. For this purpose the detector device is equipped with an ultrasound sensor consisting of an ultrasound transducer 1 and an ultrasound reflector 2. The ultrasound transducer and the ultrasound reflector can be a combined device with a piezoelectric element cast in a housing as illustrated in fig. 1. The absorbent body 3, e.g. in the form of an "ERGON Sorb" cloth, is placed either around the transducer 1 or as shown here, between the transducer 1 and the reflector 2. An acoustic jelly 4 is provided between the transducer 1 and the absorbent body 3 in order to improve the acoustic coupling between the transducer 1 and the absorbent body or cloth 3. When the cloth 3 is not moistened with oil, its ultrasound transmissivity is very poor and consequently the ultrasound signal will not penetrate the cloth. If the cloth 3 is moistened with oil, it has good ultrasound transmissivity and a signal transmitted from the transducer 1 will penetrate the cloth 3, be reflected from the reflector 2 and in turn be detected by the transducer 1. It should be understood that the detector device also may be designed with a transmitter in the form of an ultrasound transducer together with a receiver in the form of a second ultrasound transducer, the transmitter and the receiver respectively being provided on opposite sides of the absorbent body 3, i.e. the "ERGON Sorb" cloth.

The detector device based on an ultrasound sensor is robust and provides a good signal-noise ratio by using the pulse echo technique. A disadvantage of using an ultrasound sensor in a detector device is that it can only be used in water, while at the same time a certain amount of electronics are required for pulse generation and detection. Moreover, the arrangement of the transducer and the reflector is critical with a view to obtaining a good echo, nor should there be any bubbles in the water. However, an increase in pulse level not only provides a stronger echo, but also makes the transducer's position in relation to the reflector less critical.

A second sensor for use in the detector device according to another embodiment is shown in fig. 2. This sensor is based on an alteration in the optical reflectance of the absorbent body 3 when the body is moistened with, e.g., oil. When an absorbent body 3 in the form of an "ERGON Sorb" cloth is used, this cloth is basically white and is a very good reflector of light. The difference in reflectance with a moistened and an unmoistened cloth will be dependent on the colour of the oil. It is obvious that a black oil like unrefined oil, waste oil or the like will cause a greater alteration in the reflectance than a clear oil. In the detection of the optical reflectance a sensor is used based on an optical transmitter 6, e.g. a photo-diode, and an optical receiver 7, e.g. a phototransistor. The transmitter and the receiver 7 can be installed in the same plane in a sensor housing 5 and shielded from direct light between transmitter and receiver. In connection with the optical transmitter 6 electronics,will be provided which may cause a modulation of the light, a feature which is well known in the art. The measurement of reflected light is performed via the phototransistor and the results of the measurement are referred to the measurement conducted with a dry absorbent body, i.e. an unmoistened sheet. In purely practical terms the detector device in this case can be designed in such a way that the optical transmitter 6 and receiver 7 are provided in the same plane in the sensor housing 5 which is open at one end, but with the opening covered by the absorbent body 3, e.g. in the form of a piece of white "ERGON Sorb" cloth.

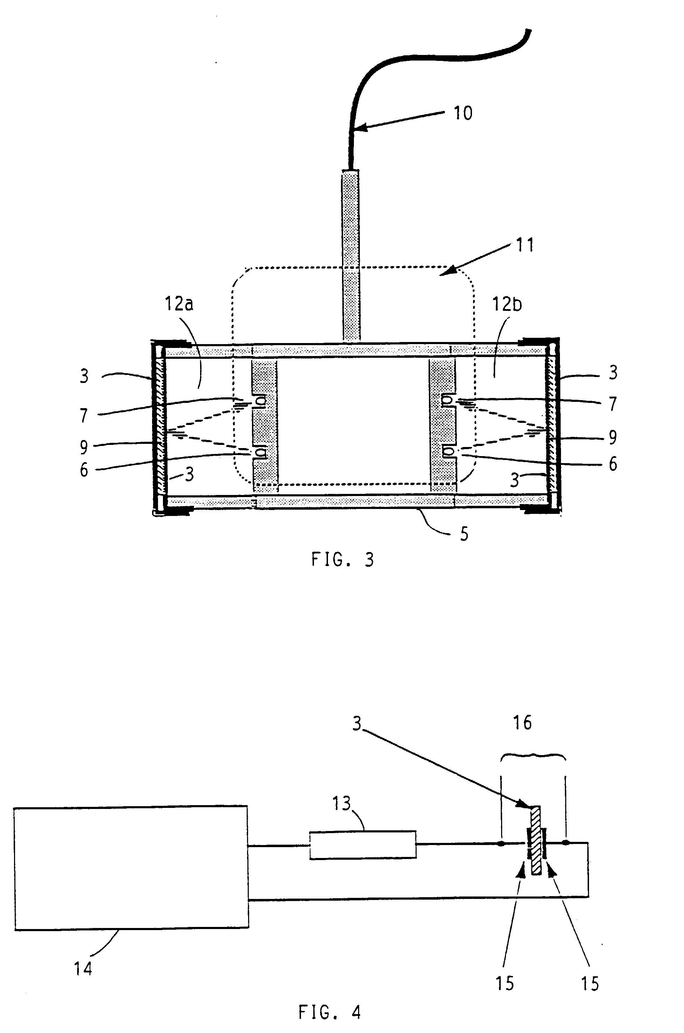

As illustrated in fig. 3, the detector device can also be equipped with an optical sensor with two transmitters 6 and receivers 7 respectively, provided in a respective chamber 12a,b in the detector housing 5, and connected to a supply cable 10, an opening of the chamber being covered in turn by the absorbent body 3 and the detector designed in such a way that the absorbent body will be in contact with the surface of the liquid, for instance by providing a buoyance block 11, if the detector is intended for use in water. In this case the respective transceiver pair 6, 7 can be arranged to perform the measurement in different wavelength frequencies. One transceiver 6, 7, for example, can perform a measurement in the UV range, while the other can perform a measurement in the IR range. This can be expedient if the absorbed hydrocarbon has a different reflectance in the two wavelength frequencies. Thus the measurements from each of the transceiver pairs can be appropriately correlated in order to provide a more reliable detection. The measurements can naturally also be conducted in the wavelength frequency for visible light and there is nothing to prevent an additional pair of transceivers from being used.

If the liquid hydrocarbon is colourless, i.e. clear, the reflectance value referred to that for an unmoistened cloth will show little alteration. In order to provide a more reliable detection the absorbent body 3, i.e. the "ERGON Sorb" cloth can therefore be impregnated with a hydrocarbon-soluble dye 13. In practical terms this can be done be providing the absorbent body 3 with a dye-impregnated intermediate layer 9. If it becomes moistened with a liquid hydrocarbon such as oil, the hydrocarbon penetrates into the intermediate layer 9 and dissolves the dye, thus adding colour to the absorbent body 3 and providing a greater alteration in the reflectance signal.

Experiment shows that a detector device based on an optical sensor provides a simple and robust solution. The necessary electronics, which are otherwise well known to those skilled in the art, are also simple as well as inexpensive and in addition the measurement results show a satisfactory difference between a dry and a moistened absorbent body.

A third embodiment of the detector device according to the invention can be based on the measurement of the dielectric constant for the absorbent body 3. There will be a difference in the dielectric constant for a dry and a moistened absorbent body 3. Oil, e.g., has a relative dielectric constant of 2-3. If the "ERGON Sorb" cloth is used as the absorbent body, it can be assumed that it has the same dielectric constant as air, i.e. a relative dielectric constant of 1. This means that the absorbent body's dielectric constant will alter when it is moistened with oil. In purely practical terms the detector device in this case can be equipped with a sensor as illustrated in fig. 4. The actual sensor consists of a plate condenser 4 with the absorbent body 3 as dielectric medium. The plate condenser 15 is provided in series with an ohmic resistance 13 across the input and output of an oscillator and similarly in series between the input and output of a not shown voltmeter device, the condenser 15 thus being located in a parallel branch 16 between the oscillator 14 and the voltmeter device. The alteration in condenser voltage, i.e. in the condenser's capacitance, will be a measure of the alteration which is obtained in the dielectric constant when the absorbent body 3 is moistened or dry. The problem with a detector device based on the measurement of the dielectric constant, however, is that first of all the detection sensitivity is slight and even only a low absorption of water in the absorbent body 3 will probably lead to a relatively substantial alteration in the measurement values, since the dielectric constant for water is approximately 80 times that for air. The advantage of a detector device according to the invention, based on the measurement of the dielectric constant, is that it only requires very simple electronics.

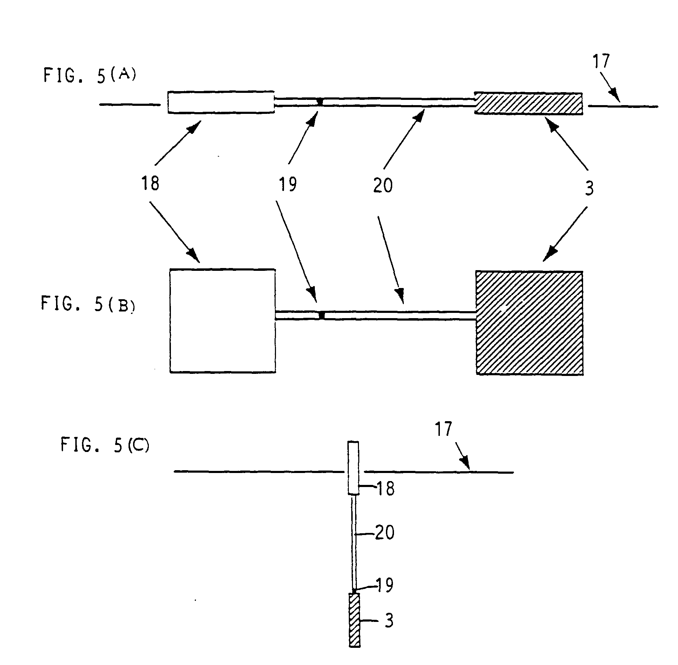

If the detector device according to the invention has to be used on water, the detection of discharge of hydrocarbons can employ an alteration in the absorbent body's buoyancy. If an "ERGON Sorb" cloth is used as the absorbent body, it will basically have a low specific weight and float easily on the water. At the same time the cloth naturally attracts very little water, but can absorb a large amount of oil, thus causing the buoyancy to alter radically after the absorption of oil. This can be used in a detector device where the sensor is designed as a buoyancy sensor, as illustrated in fig. 5 which shows the sensor in elevation in (A) and (C) and from above in (B). The detector as shown in the figure is designed as a floating body 18 which is connected via a pipe 20 to the absorbent body 3 in the form of a piece of "ERGON Sorb" cloth. Inside the pipe 20 there is provided a ball 19 which can move freely. The detector device is arranged in such a way that it will float on the surface 17 of the water (Fig. 5(A)). After the cloth 3 has absorbed the floating hydrocarbon or oil, it will sink. The ball 19 in the pipe 20 is intended to provide a hysteresis effect, but, after a certain amount of oil has been absorbed in the absorbent body 3, it will roll through the pipe 20 towards this and cause the detector to change position, e.g. so that the floating body 18 is tipped up (Fig. 5(C)), giving a visual indication of the oil discharge. The alteration in the position of the detector by means of a change in the buoyancy of the absorbent body 3 can also be indicated by its being detected by a switch mechanism which triggers an electrical signal which is transferred to a receiver station. The electrical signal may also trigger a flashing light mounted on the detector device, or trigger an acoustic signal or activate a radio direction finder.

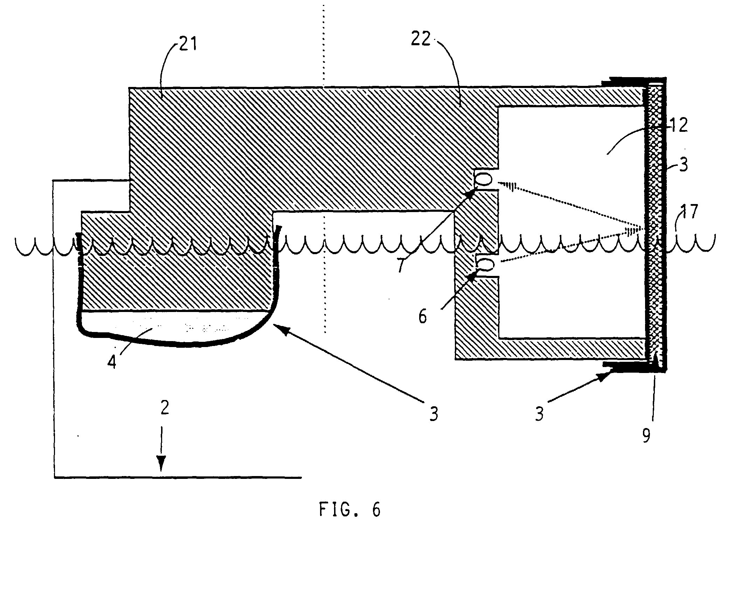

It should be understood that the different sensor types described here with a view to using them in the detector device can be used separately or combined in an integrated detector device. Thus the detector device can, e.g., be based both on an ultrasound sensor 21 and an optical sensor 22, as illustrated in fig. 6, wherein the reference numbers denote parts similar to those in Figs. 2 and 3, and the signals from the two sensors can be correlated in order to provide a more reliable indication of the detection of discharge of hydrocarbons such as oil.

If the method and detector device are to be used in the detection of hydrocarbon discharge on water, the detector device can be designed as a small buoy and possibly be anchored at a suitable spot.

Designed in this way, it will be possible to deploy the detector device according to the invention in great numbers and use them to monitor shipping lanes and oil terminals where there is a high risk of discharge of hydrocarbons. The detection signals can then be transferred from each individual detector via a cable system to a central monitoring unit or they may also be transferred by means of a miniaturized radio transmitter provided in the detector. Free-floating detectors can be designed as floating buoys and equipped with a radio direction finder. In this case the direction finder will be capable of transmitting a signal automatically when a discharge is detected, thus enabling the position of the discharge to be determined.

When the,detector device is used in the water, it is of course based on the assumption that it must be designed in such a manner that the absorbent body, which itself is basically buoyant, is situated on or near the surface of the water where the discharge of the liquid hydrocarbon will be located. There are a number of ways in which the detector device can be designed in practice, in order to ensure that the absorbent body absorbs discharge on the surface of the water.

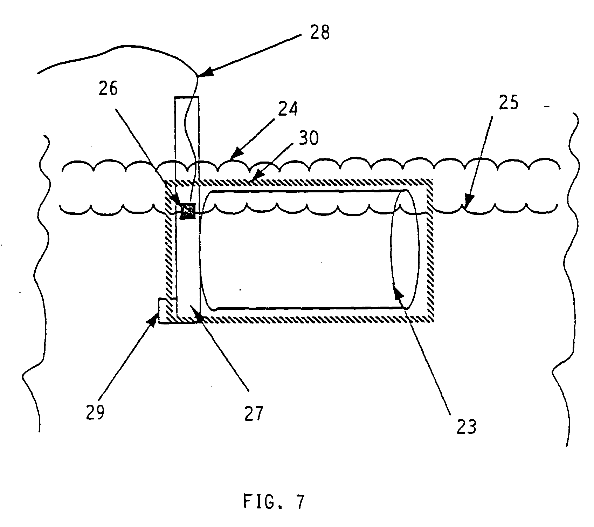

The method and the detector device according to the present invention can naturally also be used for the detection of the discharge of hydrocarbons on the ground, e.g. in the form of leakage from tanks, pipelines or transport vehicles. This is again based on the assumption that the detector device is so positioned and with the absorbent body arranged so as to enable absorption of the discharge to take place and reliable detection to be achieved. Similarly the detector device can be located in connection with buried oil tanks or the like and detect a leakage from the oil tank as illustrated schematically in fig. 7, showing the oil tank 23 burried beneath the surface level 24 and below the ground water level 25. The detector 26 is provided in a detector tube 27 at the ground water level 25 and connected to signal cable 28, the detector tube 27 providing a passage between the detector 26 and an oil trap 29. The whole arrangement with the tank is enclosed in tarpaulin 30.

Finally the detector can also be used for the detection of oil pollution in the ground water in which case it naturally does not need to be located in the vicinity of the pollution source. On the contrary, such buried detectors connected with a monitoring system can be used for rubbish dumps, industrial sites and the like where the possibility exists of a leakage of liquid hydrocarbons into the subsurface. By using a network of such buried detector devices and recording the times of detection, it will be possible to localize the source of pollution.

In a more comprehensive system for monitoring the discharge of liquid hydrocarbons and oil spills, it will naturally be expedient to be able to connect a number of detector devices to a central monitoring unit, which devices are arranged so as to transmit detection signals to the monitoring unit. This transmission can be performed automatically or by the central monitoring unit polling the individual detectors in the system according to a set plan.

## Claims

1. A method for the detection of hydrocarbons present in liquid form at normal ambient temperature, the method suitable for detection in water, on the ground or in the subsurface, especially for the detection of oil spillage on water or in ground water, said method comprising:
providing a detector having an absorbent body (3) comprising a polypropylene material, which demonstrates a selective, passive absorption of liquid hydrocarbons, especially mineral oil, which is about 487 times greater than that for water.
placing said detector in a place where hydrocarbon discharge may occur, and
detecting a change in one or more physical parameters distinguishing said absorbent body (3),
wherein said detection includes either separately or in combination the detection of an alteration in the absorbent body's ultrasound transmissivity, an alteration in the absorbent body's optical reflectance, an alteration in the absorbent body's dielectric constant, and an alteration in the absorbent body's buoyancy in water.

2. A method according to claim 1, characterized in that said polypropylene material is in the form of a non-woven cloth.

3. A method according to Claim 2, characterized in that in order to detect the alteration in an individual physical parameter a sensor (1,2; 6,7; 16; 18) is used which is arranged to detect the respective physical parameter.

4. A method according to Claim 3, characterized in that two or more of the individual sensors (1,2; 6,7; 16; 18) arranged to detect the respective physical parameter are combined in an integrated detector device (21,22).

5. A method according to one of the preceding Claims characterized in that for the detection of an alteration in the absorbent body's ultrasound transmissivity a sensor is used with an ultrasound transmitter (1) and an ultrasound receiver (2) provided respectively on each side of the absorbent body (3) or a sensor with the absorbent body provided between a combined ultrasound transceiver and an ultrasound reflector.

6. A method according to one of the Claims 1-4, characterized in that for the detection of the absorbent body's optical reflectance a sensor is used with an optical transmitter (6) and an optical receiver (7) provided respectively beside the absorbent body (3), so that an optical signal transmitted by the transmitter (6) is reflected by the absorbent body (3) back to the receiver (7).

7. A method according to Claim 6, characterized in that in the absorbent body (3) there is provided a dye which is dissolved in hydrocarbons in liquid form and causes a further alteration in the body's optical reflectance.

8. A method according to Claim 7, characterized in that the optical reflectance is measured in the UV range and/or in the visible range and/or in the IR range.

9. A method according to one of the Claims 1-4, characterized in that for the detection of the absorbent body's dielectric constant a sensor (16) is used which is arranged so as to measure the energy which is exchanged between a surrounding medium and an oscillator.

10. A method according to one of the Claims 1-4, characterized in that for the detection of the absorbent body's buoyancy in the water a sensor is used with a floating body (18) connected to the absorbent body (3) a buoyancy indicator provided in the sensor being activated by an alteration in the absorbent body's buoyancy.

11. A detector device for performing the method according to any one of the Claims 1-10, the detector device comprising
an absorbent body (3) formed by a polypropylene material which demonstrates a selective, passive absorption of liquid hydrocarbons, especially mineral oil, which is about 487 times greater than that for water and one or more sensors (1,2; 6,7; 16; 18) provided in connection with the absorbent body (3) and arranged for the detection of an alteration in one or more physical parameters distinguishing the absorbent body,
the sensor or sensors (1,2; 6,7) being one or more of the group of an ultrasound sensor (1,2), an optical sensor (6,7), a sensor for the dielectric constant (16) and a buoyancy sensor (18).

12. A detector device according to Claim 11, characterized in that the detector device comprises combinations of two or more sensors (1,2; 6,7; 16; 18) arranged for the detection of two or more of the respective physical parameters.

13. A detector device according to Claim 11, characterized in that the ultrasound sensor comprises respectively an ultrasound transmitter (1) and an ultrasound receiver (2) provided on each side of the absorbent body (3), or with the absorbent body (3) provided between a ultrasound transducer (1) and an ultrasound reflector (2).

14. A detector device according to Claim 11, characterized in that the optical sensor comprises respectively an optical transmitter (6) and an optical receiver (7) provided beside the absorbent body (3), thus providing a free optical path between the transmitter, one of the absorbent body's surfaces and the receiver.

15. A detector device according to Claim 14, characterized in that in the absorbent body (3) there is provided a dye (9) which is arranged to be dissolved in hydrocarbons in liquid form.

16. A detector device according to Claim 14, characterized in that the optical sensor is a UV sensor or a sensor for the visible light range or an IR sensor.

17. A detector device according to Claim 16, characterized in that the optical sensor is equipped with at least two optical transmitters (6) and at least two optical receivers (7), assigned to the respective transmitter, the transceiver pairs (6,7) being arranged so as to measure the optical reflectance in the same wavelength frequency or each in its own wavelength frequency.

18. A detector device according to Claim 11, characterized in that the sensor for the dielectric constant comprises a plate condenser (15) with the absorbent body (3) as a dielectric medium, the plate condenser (15) being connected in series with an oscillator (14) and also with the clamps on a voltmeter device, in such a manner that the measured condenser voltage becomes an expression of the absorbent body's dielectric constant.

19. A detector device according to Claim 11, characterized in that the buoyancy sensor comprises a floating body (18) connected with the absorbent body (3) and a buoyancy indicator provided in the sensor and arranged to be activated by an alteration in the absorbent body's buoyancy, the buoyancy indicator either being arranged to provide a visual indication and/or indication in the form of an electrical signal in reaction to an alteration in the absorbent body's buoyancy.

20. A detector device according to one of the Claims 11-19, characterized in that the detector device is arranged to be connected with a central monitoring unit.

21. A detector device according to the preceding Claim, characterized in that it comprises means for the transfer of detection signals from one or more of the sensors to a monitoring unit, either automatically or in reaction to a poll from the central monitoring unit.

## Patentansprüche

1. Verfahren zum Nachweis von Kohlenwasserstoffen, die in flüssiger Form bei normaler Umgebungstemperatur anwesend sind, wobei das Verfahren geeignet ist zum Nachweis in Wasser, an der Erdoberfläche oder unter der Oberfläche, insbesondere zum Nachweis der Freisetzung von Öl auf Wasser oder im Grundwasser, wobei das Verfahren folgendes aufweist:
Vorsehen eines Detektors, der einen absorptionsfähigen Körper (3) hat, der ein Polypropylenmaterial aufweist, das eine selektive, passive Absorption für flüssige Kohlenwasserstoffe, insbesondere Mineralöl, zeigt, die ungefähr 487mal größer als diejenige für Wasser ist,
Anordnen des Detektors an einem Ort, an dem ein Austritt von Kohlenwasserstoff stattfinden kann, und
Messen einer Änderung eines oder mehrerer physikalischer Parameter, die für den absorptionsfähige Körper (3) charakteristisch sind,
wobei diese Messung entweder getrennt oder in Kombination aufweist: die Messung einer Änderung des Ultraschall-Transmissionsgrads des absorptionsfähigen Körpers, einer Änderung des optischen Reflexionsvermögens des absorptionsfähigen Körpers, einer Änderung der Dielektrizitätskonstanten des absorptionsfähigen Körpers, und einer Änderung des Auftriebs des absorptionsfähigen Körpers in Wasser.

2. Verfahren nach Anspruch 1, dadurch gekennzeichnet, daß das Polypropylenmaterial in Form eines Faservliesstoffs ist.

3. Verfahren nach Anspruch 2, dadurch gekennzeichnet, daß zum Messen der Änderung eines einzelnen physikalischen Parameters ein Sensor (1, 2; 6, 7; 16; 18) verwendet wird, der angeordnet ist, um den jeweiligen physikalischen Parameter zu erfassen.

4. Verfahren nach Anspruch 3, dadurch gekennzeichnet, daß zwei oder mehr der einzelnen Sensoren (1, 2; 6, 7; 16; 18), die angeordnet sind, um den jeweiligen physikalischen Parameter zu erfassen, in einer integrierten Detektoreinrichtung (21, 22) kombiniert sind.

5. Verfahren nach einem der vorhergehenden Ansprüche, dadurch gekennzeichnet, daß zum Messen einer Änderung des Ultraschall-Transmissionsgrads des absorptionsfähigen Körpers ein Sensor mit einem Ultraschallsender (1) und einem Ultraschallempfänger (2), die jeweils auf einer Seite des absorptionsfähigen Körpers (3) vorgesehen sind, oder ein Sensor verwendet wird, bei dem der absorptionsfähige Körper zwischen einem kombinierten Ultraschall-Sende-Empfänger und einem Ultraschall-Reflektor vorgesehen ist.

6. Verfahren nach einem der Ansprüche 1 bis 4, dadurch gekennzeichnet, daß zum Messen des optischen Reflexionsvermögens des absorptionsfähigen Körpers ein Sensor mit einem optischen Sender (6) und einem optischen Empfänger (7) verwendet wird, die jeweils neben dem absorptionsfähigen Körper (3) vorgesehen sind, so daß ein von dem Sender (6) ausgehendes optisches Signal von dem absorptionsfähigen Körper (3) zu dem Empfänger (7) zurückreflektiert wird.

7. Verfahren nach Anspruch 6, dadurch gekennzeichnet, daß in dem absorptionsfähigen Körper (3) ein Farbstoff vorgesehen ist, der sich in Kohlenwasserstoffen in Flüssigform löst und eine weitere Änderung des optischen Reflexionsvermögens des Körpers verursacht.

8. Verfahren nach Anspruch 7, dadurch gekennzeichnet, daß das optische Reflexionsvermögen im UV-Bereich und/oder im sichtbaren Bereich und/oder im IR-Bereich gemessen wird.

9. Verfahren nach einem der Ansprüche 1 bis 4, dadurch gekennzeichnet, daß zum Messen der Dielektrizitätskonstanten des absorptionsfähigen Körpers ein Sensor (16) verwendet wird, der so angeordnet ist, daß er die Energie mißt, die zwischen einem umgebenden Medium und einem Oszillator ausgetauscht wird.

10. Verfahren nach einem der Ansprüche 1 bis 4, dadurch gekennzeichnet, daß zum Messen des Auftriebs des absorptionsfähigen Körpers im Wasser ein Sensor mit einem Schwimmerkörper (18), der mit dem absorptionsfähigen Körper (3) verbunden ist, verwendet wird, wobei ein in dem Sensor vorgesehener Auftriebsindikator durch eine Änderung des Auftriebs des absorptionsfähigen Körpers aktiviert wird.

11. Detektoreinrichtung zur Durchführung des Verfahrens nach einem der Ansprüche 1 bis 10, wobei die Detektoreinrichtung folgendes aufweist:
einen absorptionsfähigen Körper (3), der aus einem Polypropylenmaterial gebildet ist, das eine selektive, passive Absorption für flüssige Kohlenwasserstoffe, insbesondere Mineralöl, zeigt, die ungefähr 487mal größer als diejenige für Wasser ist, und ein oder mehr Sensoren (1, 2; 6, 7; 16; 18), die in Verbindung mit dem absorptionsfähigen Körper (3) vorgesehen und angeordnet sind, um eine Änderung eines oder mehrerer physikalischer Parameter zu messen, die für den absorptionsfähigen Körper charakteristisch sind,
wobei der Sensor oder die Sensoren (1, 2; 6, 7) einer oder mehrere der Gruppe aus einem Ultraschallsensor (1, 2), einem optischen Sensor (6, 7), einem Sensor (16) für die Dielektrizitätskonstante und einem Auftriebssensor (18) sind.

12. Detektoreinrichtung nach Anspruch 11, dadurch gekennzeichnet, daß die Detektoreinrichtung Kombinationen aus zwei oder mehr Sensoren (1, 2; 6, 7; 16; 18) aufweist, die zum Messen von zwei oder mehr der jeweiligen physikalischen Parameter angeordnet sind.

13. Detektoreinrichtung nach Anspruch 11, dadurch gekennzeichnet, daß der Ultraschallsensor einen Ultraschallsender (1) und einen Ultraschallempfänger (2) aufweist, die an jeder Seite des absorptionsfähigen Körpers (3) vorgesehen sind, oder daß der absorptionsfähige Körper (3) zwischen einem Ultraschallwandler (1) und einem Ultraschallreflektor (2) vorgesehen ist.

14. Detektoreinrichtung nach Anspruch 11, dadurch gekennzeichnet, daß der optische Sensor einen optischen Sender (6) und einen optischen Empfänger (7) aufweist, die neben dem absorptionsfähigen Körper (3) so vorgesehen sind, daß zwischen dem Sender, einer der Oberflächen des absorptionsfähigen Körpers und dem Empfänger ein freier optischer Weg geschaffen ist.

15. Detektoreinrichtung nach Anspruch 14, dadurch gekennzeichnet, daß in dem absorptionsfähigen Körper (3) ein Farbstoff (9) vorgesehen ist, der angeordnet ist, um in Kohlenwasserstoffen in Flüssigform gelöst zu werden.

16. Detektoreinrichtung nach Anspruch 14, dadurch gekennzeichnet, daß der optische Sensor ein UV-Sensor oder ein Sensor für den Bereich des sichtbaren Lichts oder ein IR-Sensor ist.

17. Detektoreinrichtung nach Anspruch 16, dadurch gekennzeichnet, daß der optische Sensor mit wenigstens zwei optischen Sendern (6) und wenigstens zwei optischen Empfängern (7), die dem jeweiligen Sender zugeordnet sind, ausgestattet ist, wobei die Sender-Empfänger-Paare (6, 7) so angeordnet sind, daß sie das optische Reflexionsvermögen in derselben Wellenlängenfrequenz oder jeweils in ihrer eigenen Wellenlängenfrequenz messen.

18. Detektoreinrichtung nach Anspruch 11, dadurch gekennzeichnet, daß der Sensor für die Dielektrizitätskonstante einen Plattenkondensator (15) mit dem absorptionsfähigen Körper (3) als einem dielektrischen Medium aufweist, wobei der Plattenkondensator (15) in Reihe mit einem Oszillator (14) und außerdem mit den Klemmen an einem Spannungsmeßgerät verbunden ist derart, daß die gemessene Kondensatorspannung ein Ausdruck der Dielektrizitätskonstanten des absorptionsfähigen Körpers wird.

19. Detektoreinrichtung nach Anspruch 11, dadurch gekennzeichnet, daß der Auftriebssensor einen mit dem absorptionsfähigen Körper (3) verbundenen Schwimmerkörper (18) sowie einen Auftriebsindikator aufweist, der in dem Sensor vorgesehen und angeordnet ist, um von einer Änderung des Auftriebs des absorptionsfähigen Körpers aktiviert zu werden, wobei der Auftriebsindikator angeordnet ist, um entweder eine Sichtanzeige und/oder eine Anzeige in Form eines elektrischen Signals als Reaktion auf eine Änderung des Auftriebs des absorptionsfähigen Körpers zu liefern.

20. Detektoreinrichtung nach einem der Ansprüche 11 bis 19, dadurch gekennzeichnet, daß die Detektoreinrichtung angeordnet ist, um an eine zentrale Überwachungseinheit angeschlossen zu werden.

21. Detektoreinrichtung nach dem vorhergehenden Anspruch, dadurch gekennzeichnet, daß sie Mittel aufweist, um Meßsignale von einem oder mehreren der Sensoren an eine Überwachungseinheit zu übertragen, und zwar entweder automatisch oder in Abhängigkeit von einer Abfrage durch die zentrale Überwachungseinheit.

## Revendications

1. Méthode pour la détection d'hydrocarbures présents sous forme liquide à température ambiante normale, utilisable pour la détection dans l'eau, sur le sol ou dans le sol, en particulier pour la détection de la pollution par le pétrole de l'eau ou de l'eau dans le sol, la méthode comprenant les étapes consistant à :
utiliser un détecteur ayant un corps absorbant (3) comprenant un matériau en polypropylène qui possède une absorption sélective et passive pour les hydrocarbures liquides, en particulier le pétrole, qui est environ 487 fois plus grande que celle pour l'eau,
mettre le détecteur en place quand une fuite d'hydrocarbure se produit, et
détecter un changement dans un ou plusieurs paramètres physiques en relation avec le corps absorbant (3),
dans laquelle la détection comprend, soit séparément, soit en combinaison, la détection d'une modification dans le corps absorbant de la transmissivité ultrasonique, une modification dans le corps absorbant de la réflectance optique, une modification dans le corps absorbant de la constante diélectrique, et une modification dans le corps absorbant de la flottabilité dans l'eau.

2. Méthode selon la revendication 1, caractérisée en ce que le matériau en polypropylène est sous forme d'un tissu non-tissé.

3. Méthode selon la revendication 2, caractérisée en ce que, pour détecter la modification d'un paramètre physique individuel, on utilise un capteur (1,2;6,7;16;18) qui est mis en place pour détecter le paramètre physique respectif.

4. Méthode selon la revendication 3, caractérisée en ce que deux ou plus de deux capteurs individuels (1,2;6,7;16;18), mis en place pour détecter les paramètres physiques respectifs, sont combinés dans un dispositif détecteur intégré (21,22).

5. Méthode selon l'une des revendications précédentes, caractérisée en ce que, pour détecter la modification dans le corps absorbant de la transmissivité ultrasonique, on utilise un capteur comprenant un émetteur ultrasonique (1) et un récepteur ultrasonique (2) placés respectivement de chaque côté du corps absorbant (3) ou un capteur où le corps absorbant est placé entre un émetteur ultrasonique et un récepteur ultrasonique combinés.

6. Méthode selon l'une des revendications 1 à 4, caractérisée en ce que, pour détecter la réflectance optique du corps absorbant, on utilise un capteur comprenant un émetteur optique (6) et un récepteur optique (7) placés respectivement à côté du corps absorbant (3), en sorte que le signal optique transmis par l'émetteur (6) est réfléchi par le corps absorbant (3) vers le récepteur (7).

7. Méthode selon la revendication 6, caractérisée en ce que dans le corps absorbant (3) se trouve un colorant qui se dissout dans les hydrocarbures sous forme liquide et provoque une modification supplémentaire dans la réflectance optique du corps.

8. Méthode selon la revendication 7, caractérisée en ce que la réflectance optique est mesurée dans la zone UV et/ou dans la zone visible et/ou dans la zone IR.

9. Méthode selon l'une des revendications 1 à 4, caractérisée en ce que, pour détecter la constante diélectrique du corps absorbant, on utilise un capteur (16) qui est placé de façon à mesurer l'énergie qui est échangée entre le milieu environnant et un oscillateur.

10. Méthode selon l'une des revendications 1 à 4, caractérisée en ce que, pour détecter la flottabilité du corps absorbant dans l'eau, on utilise un capteur comprenant un corps flottant (18) relié au corps absorbant (3), un indicateur de flottabilité prévu dans le capteur étant activé par la modification de la flottabilité du corps absorbant.

11. Dispositif détecteur pour la mise en oeuvre de la méthode selon l'une des revendications 1 à 10, comprenant
un corps absorbant (3) en matériau polypropylène qui possède une absorption sélective et passive pour les hydrocarbures liquides, en particulier le pétrole, qui est environ 487 fois plus grande que celle pour l'eau et un ou plusieurs capteurs (1,2;6,7;16;18) prévus en relation avec le corps absorbant (3) et mis en place pour détecter une modification d'un ou plusieurs paramètres physiques caractérisant le corps absorbant,
le ou les capteurs (1,2;6,7) appartenant à l'un au moins des groupes comprenant un capteur ultrasonique (1,2), un capteur optique (6,7), un capteur de constante diélectrique (16) et un capteur de flottabilité (18).

12. Dispositif détecteur selon la revendication 11, caractérisé en ce que le dispositif détecteur comprend des combinaisons de deux ou plus de deux capteurs (1,2;6,7;16;18) mis en place pour la détection de deux ou plus de deux de paramètres physiques respectifs.

13. Dispositif détecteur selon la revendication 11, caractérisé en ce que le capteur ultrasonique comprend respectivement un émetteur ultrasonique (1) et un récepteur ultrasonique (2) placés de chaque côté du corps absorbant (3), ou dans lequel le corps absorbant (3) est placé entre un transducteur ultrasonique (1) et un réflecteur ultrasonique (2).

14. Dispositif détecteur selon la revendication 11, caractérisé en ce que le capteur optique comprend respectivement un émetteur optique (6) et un récepteur optique (7) placés à côté du corps absorbant (3), créant ainsi un chemin optique libre entre l'émetteur, une des surfaces du corps absorbant et le récepteur.

15. Dispositif détecteur selon la revendication 14, caractérisé en ce que le corps absorbant (3) comprend un colorant (9) qui est destiné à se dissoudre dans les hydrocarbures sous forme liquide.

16. Dispositif détecteur selon la revendication 14, caractérisé en ce que le capteur optique est un capteur UV ou un capteur de lumière visible ou un capteur IR.

17. Dispositif détecteur selon la revendication 16, caractérisé en ce que le capteur optique est équipé d'au moins deux émetteurs optiques (6) et au moins deux récepteurs optiques (7), chacun pour un émetteur, les paires d'émetteurs-récepteurs (6,7) étant conçues pour mesurer la réflectance optique dans la même fréquence de longueurs d'onde ou chacun dans sa propre fréquence de longueurs d'onde.

18. Dispositif détecteur selon la revendication 11, caractérisé en ce que le capteur pour la constante diélectrique comprend un condensateur à plaque (15) ayant le corps absorbant (3) comme milieu diélectrique, le condensateur à plaque (15) étant relié en série à un oscillateur (14) et également aux bornes d'un dispositif voltamètre, de telle façon que le voltage mesuré sur le condensateur devienne une expression de la constante diélectrique du corps absorbant.

19. Dispositif détecteur selon la revendication 11, caractérisé en ce que le capteur de flottabilité comprend un corps flottant (18) relié au corps absorbant (3) et un indicateur de flottabilité dans le capteur et conçu pour être activé par une modification dans la flottabilité du corps absorbant, l'indicateur de flottabilité étant conçu pour fournir une indication visuelle et/ou une indication sous la forme d'un signal électrique en réaction à la modification dans la flottabilité du corps absorbant.

20. Dispositif détecteur selon l'une des revendications 11 à 19, caractérisé en ce qu'il est conçu pour être connecté à une unité centrale de surveillance.

21. Dispositif détecteur selon l'une des revendications précédentes, caractérisé en ce qu'il comprend des moyens pour le transfert et la détection des signaux d'un ou plusieurs des capteurs vers une unité de surveillance, soit automatiquement, soit en réaction à un appel de l'unité centrale de surveillance.
